# EUROPÄISCHE PATENTSCHRIFT

(11) **EP 1 234 815 B1**
(45) Veröffentlichungstag und Bekanntmachung des Hinweises auf die Patenterteilung: **07.03.2007**
(21) Anmeldenummer: 02002614.2
(22) Anmeldetag: 05.02.2002
(51) Int. Cl.: C07C 215/40, C07D 307/32

(54) **Verfahren zur Herstellung von kristallinem Cholinascorbat**
Process for the manufacture of crystalline choline ascorbate
Procédé pour l'obtention d' ascorbate de choline cristallin

(30) Priorität: 23.02.2001 DE 10109073
(43) Veröffentlichungstag der Anmeldung: 28.08.2002
(73) Patentinhaber: BASF Aktiengesellschaft, 67056 Ludwigshafen (DE)
(72) Erfinder: Völkel, Ludwig, 67117 Limburgerhof (DE); Oftring, Alfred, Dr., 67098 Bad Dürkheim (DE); Hasselwander, Oliver, Dr., 76829 Landau (DE); Sindel, Ulrike, Dr., 80335 München (DE); Krämer, Klaus, Dr., 76829 Landau (DE)

(56) Entgegenhaltungen:
- EP-A- 0 812 821
- CH-A- 490 322
- FR-A- 1 242 805
- US-A- 2 823 166
- US-A- 4 394 377
- KAVALALI, GULSEL ET AL: "Isolation of choline ascorbate from Apium graveolens" JOURNAL OF NATURAL PRODUCTS, Bd. 48, Nr. 3, 1985, Seite 495 XP002260785

## Beschreibung

Die Erfindung betrifft ein Verfahren zur Herstellung von kristallinem Cholinascorbat.

Cholin {[(H₃C)₃N⁺-CH₂-CH₂-OH]OH⁻]} ist der basische Bestandteil der Phospholipide vom Phosphoglyceridtyp und im Pflanzen- und Tierreich weit verbreitet. Cholin fungiert als wichtiger Faktor bei biochemischen Prozessen, z.B. bei Methylierungen. Sein Mangel führt bei Tieren zur Bildung der Fettleber.

Cholin wird hauptsächlich in Form von Cholinchlorid oder Cholinbitartrat in Arzneipräparaten gegen Arterienverkalkung und Leberparenchymschäden eingesetzt. In der Tierernährung stellt Cholinchlorid einen bedeutenden Futtermittelzusatzstoff dar.

Cholinsalze organischer Säuren, wie z.B. das oben genannte Cholinbitartrat, oder Cholinsalicylat, Cholinhydrogencitrat sowie Cholinascorbat werden u.a. beschrieben in EP-A-0 812 821.

Die Synthese von Cholinascorbat ist Gegenstand von US 2,823,166 und CH 490322. Die in diesen Patenschriften beschriebenen Herstellverfahren liefern jedoch ein Cholinascorbat, das sich nur als hochviskoses Öl isolieren läßt, deren Reinheit und Stabilität für die Anwendung im Lebensmittel- und pharmazeutischen Bereich nicht immer ausreichend ist. Ferner führt die Verwendung eines solchen Öls als Tierfuttermittel oder als Zusatzstoff beispielsweise in Multivitamintabletten häufig zu anwendungstechnischen Problemen.

Es war daher die Aufgabe der vorliegenden Erfindung, ein Verfahren zur Herstellung einer stabilen und hochreinen Form von Cholinascorbat bereitzustellen, das die oben genannten Nachteile des Standes der Technik nicht aufweist.

Diese Aufgabe wurde gelöst durch die Bereitstellung eines Verfahrens zur Herstellung von kristallinem Cholinascorbat durch Umsetzung von Ascorbinsäure mit Trimethylamin und Ethylenoxid, dadurch gekennzeichnet, dass die Reaktion im Temperaturbereich von 0°C bis 5°C und in Gegenwart eines mit Wasser mischbaren organischen Lösungsmittels oder einer Mischung aus Wasser und einem mit Wasser mischbaren organischen Lösungsmittel durchgeführt wird.

Die nach dem erfindungsgemäßen Verfahren hergestellten Kristalle wurden einer Röntgenbeugungsanalyse mit Cu K-alpha-Strahlung unterzogen.

Das Kristallisat weist als intensivste Linie im 2 Θ-Röntgen-Pulverdiffraktogramm im Bereich zwischen 3,40 und 4,70 Å eine Linie bei d = 3,80Å aus.

Das nach dem erfindungsgemäßen Verfahren hergestellte kristalline Cholinascorbat weist außerdem ein Intensitätsverhältnis der Beugungslinien bei d = 3,80 Å und d = 4,55 Å von mindestens 0,5, bevorzugt mindestens 0,6, besonders bevorzugt von mindestens 0,7 sowie bei d = 3,80 Å und d = 4,67 Å von mindestens 0,4, bevorzugt mindestens 0,5, besonders bevorzugt von mindestens 0,6 auf.

Neben den Beugungslinien bei d = 3,80 Å, 4,55 Å und 4,67 Å weist das Kristallisat weitere Linien bei d = 3,46 Å, 3,78 Å, 6,91 Å, 8,49 Å und 10,29 Å auf.

Die im Rahmen der Erfindung herstellbaren Cholinascorbat-Kristalle weisen eine Reinheit von > 98%, bevorzugt > 99%, besonders bevorzugt > 99,5% auf. Im Gegensatz zu dem als Öl vorliegenden Cholinascorbat sind die nach dem erfindungsgemäßen Verfahren hergestellten Kristalle nur schwach hygroskopisch.

Anhand von Partikelgrößenbestimmungen konnte gezeigt werden, daß 20 bis 100% der im Rahmen der Erfindung hergestellten Cholinascorbat-Kristalle eine Partikelgröße im Bereich von 10 bis 2000 µm, bevorzugt von 50 bis 1000 µm, besonders bevorzugt von 100 bis 800 µm, ganz besonders bevorzugt im Bereich von 100 bis 600 µm aufweisen.

Zur Bestimmung der Größenverteilung der Cholinascorbat-Kristalle eignen sich sowohl die Siebanalyse als auch die Laserbeugungsspektrometrie, letztere insbesondere zur Bestimmung der feinkörnigen Partikel. Bei den daraus erhaltenen Ergebnissen der Partikelgrößenbestimmungen handelt es sich um Volumen- und damit um Massenverteilungen.

Das kristalline Salz zeichnet sich dadurch aus, daß es zwei für die Human- und Tierernährung wichtige Wirkstoffe in einem Molekül in stabiler, hochreiner und gut bioverfügbarer Form vereint.

Das erfindungsgemäße Verfahren ist ferner dadurch gekennzeichnet, dass der Wasseranteil im Lösungsmittel, in dem die Reaktion durchgeführt wird, zwischen 0 und 50 Gew.-%, bevorzugt zwischen 0 und 10 Gew.-% liegen kann.

Als wassermischbare Lösungsmittel sind vor allem wassermischbare, thermisch stabile, flüchtige, nur Kohlenstoff, Wasserstoff und Sauerstoff enthaltene Lösungsmittel wie Alkohole, Ether, Ester, Ketone und Acetale gemeint. Bevorzugt verwendet man solche Lösungsmittel, die mindestens zu 10% wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran oder Aceton verwendet. Ganz besonders bevorzugt seien Methanol und Ethanol genannt.

Das Molverhältnis der Reaktionspartner Trimethylamin : Ascorbinsäure : Ethylenoxid liegt im Bereich von 0,9 bis 1,1 : 0,9 bis 1,1 : 0,9 bis 2,0, bevorzugt im Bereich von 1 : 1 : 1,5, besonders bevorzugt im Bereich von 1 : 1 : 1,2.

Eine besondere Ausführungsform des erfindungsgemäßen Verfahrens ist dadurch gekennzeichnet, daß die Kristallisation von Cholinascorbat in einem der oben genannten, für die Reaktion verwendeten Lösungsmittel erfolgt.

Es ist auch möglich, zunächst Trimethylamin und Ethylenoxid in einem mit Wasser mischbaren organischen Lösungsmittel oder in einer Mischung aus Wasser und einem mit Wasser mischbaren organischen Lösungsmittels bei Temperaturen im Bereich von -20°C bis 80°C, bevorzugt -10°C bis 40°C, besonders bevorzugt im Temperaturbereich von 0°C bis 30°C umzusetzen und diese Lösung anschließend durch Zugabe einer stöchiometrischen Menge an Ascorbinsäure in Cholinascorbat zu überführen und auszukristallisieren.

Als weitere mögliche Herstellvariante läßt sich auch Cholinchlorid mit Natriumascorbat in einem mit Wasser mischbaren organischen Lösungsmittel oder in einer Mischung aus Wasser und einem mit Wasser mischbaren organischen Lösungsmittels bei Temperaturen im Bereich von -20°C bis 80°C, bevorzugt -10°C bis 40°C, besonders bevorzugt im Temperaturbereich von 0°C bis 30°C zu kristallinem Cholinascorbat umsetzen. Das dabei gebildete Natriumchlorid wird vor dem Auskristallisieren des Wertproduktes abfiltriert. In Gegenwart eines basischen, Chlorid-selektiven Ionenaustauschers läßt sich darüberhinaus die Bildung des Nebenprodukts NaCl vermeiden.

Das nach dem erfindungsgemäßen Verfahren hergestellte kristalline Cholinascorbat kann zur Herstellung von Arzneimitteln, insbesondere von Präparaten zur Bekämpfung von Leberzirrhose oder anderen Lebererkrankungen, als Zusatz in Lebensmitteln, Tierfuttermitteln oder als Komponente in Nahrungsergänzungsmitteln, beispielsweise in Multivitaminpräparaten wie Tabletten oder Gelatinekapseln verwendet werden.

Der Gehalt an kristallinem Cholinascorbat sowohl in den Arzneimitteln als auch in den Nahrungsergänzungsmitteln, beispielsweise in Multivitamintabletten, kann im Bereich von 1 bis 750 mg, bevorzugt von 2 bis 450 mg, besonders bevorzugt von 5 bis 225 mg, ganz besonders bevorzugt im Bereich von 10 bis 150 mg liegen.

In Tabletten, in denen als Wirkstoff nur Cholinsacorbat enthalten ist, kann der Gehalt an Cholinascorbat im Bereich von 50 bis 1500 mg liegen.

Anhand der folgenden Beispiele soll das erfindungsgemäße Herstellverfahren von kristallinem Cholinascorbat näher erläutert werden.

### Beispiel 1

0,2 mol Trimethylamin in Methanol (25 Gew.-%ig) wurden unter Kühlung auf 0°C mit 0,2 mol Ascorbinsäure versetzt. In diese Mischung wurden 0,2 mol Ethylenoxid so eingegast, daß die Reaktionstemperatur 0-5°C nicht überstieg. Nach Reaktionsende wurde der Reaktor mit Stickstoff gespült und bei einer Temperatur zwischen 0 und 5°C weiter gerührt. Das gebildete Cholinascorbat kristallisierte aus der Reaktionsmischung aus, wurde abfiltriert, mit Methanol gewaschen und zur weiteren Aufreinigung erneut in Methanol umkristallisiert. Man erhielt farblose Kristalle in einer Ausbeute von 80% mit einem Schmelzpunkt zwischen 123,5° und 124,4°C. Mittels Elementaranalyse, ¹³C-NMR-Spektroskopie und Einkristallstrukturanalyse wurde das Kristallisat als Cholinascorbat (wasserfrei) charakterisiert.

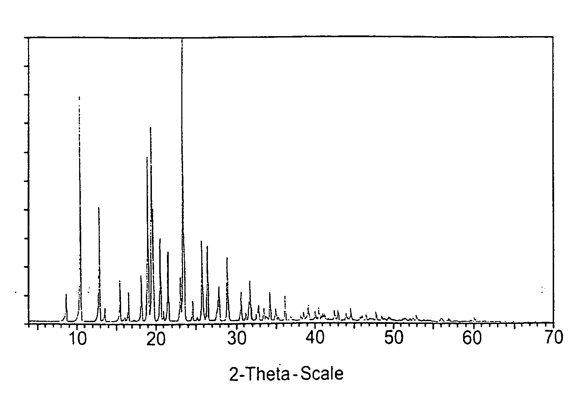

Figur 1 zeigt ein Röntgen-Pulverdiffraktogramm des nach Beispiel 1 hergestellten kristallinen Cholinascorbats (gemessen mit Siemens Difraktometer D5000, Messung in Reflexion).

### Beispiel 2

0,3 mol Trimethylamin in Methanol (25 Gew.-%ig) wurden unter Kühlung auf 0°C mit 0,3 mol Ascorbinsäure versetzt. In diese Mischung wurden 0,45 mol Ethylenoxid so eingegast, daß die Reaktionstemperatur 0-5°C nicht überstieg. Nach Reaktionsende wurde der Reaktor mit Stickstoff gespült und bei einer Temperatur zwischen 0 und 5°C weiter gerührt. Das gebildete Cholinascorbat kristallisierte aus der Reaktionsmischung aus, wurde abfiltriert, mit Methanol gewaschen und zur weiteren Aufreinigung erneut in Methanol umkristallisiert. Man erhielt farblose Kristalle in einer Ausbeute von 85% mit einem Schmelzpunkt zwischen 123,5° und 124,4°C.

### Beispiel 3

0,2 mol Trimethylamin in Methanol (25 Gew.-%ig) wurden unter Kühlung auf 0°C mit 0,2 mol Ascorbinsäure und 6 Gew.-% Wasser versetzt. In diese Mischung wurden 0,2 mol Ethylenoxid so eingegast, daß die Reaktionstemperatur 0-5°C nicht überstieg. Nach Reaktionsende wurde der Reaktor mit Stickstoff gespült und bei einer Temperatur zwischen 0 und 5°C weiter gerührt. Das gebildete Cholinascorbat kristallisierte aus der Reaktionsmischung aus, wurde abfiltriert, mit Methanol gewaschen und zur weiteren Aufreinigung erneut in Methanol umkristallisiert. Man erhielt farblose Kristalle mit einem Schmelzpunkt von 124°C.

### Beispiel 4

Multivitamintablette folgender Zusammensetzung:

| | |
|---|---|
| β-Carotin | 5 mg |
| Vitamin E | 10 mg |
| Vitamin C | 60 mg |
| Vitamin D | 1,2 mcg |
| Thiamin | 1,4 mg |
| Riboflavin | 1,6 mg |
| Pyridoxin HCl | 2,2 mg |
| Vitamin B₁₂ | 1 mcg |
| Niacin | 18 mg |
| Pantothensäure | 6 mg |
| Folsäure | 200 mcg |
| Biotin | 150 mcg |
| Cholinascorbat* | 1,2 mg |
| Magnesium | 100 mg |
| Zink | 15 mg |
| Mangan | 2,5 mg |
| Selen | 62 mcg |

| | |
|---|---|
| * hergestellt nach Beispiel 1 | |

### Beispiel 5

Multivitamintablette folgender Zusammensetzung:

| | |
|---|---|
| β-Carotin | 5 mg |
| Vitamin E | 10 mg |
| Vitamin D | 1,2 mcg |
| Thiamin | 1,4 mg |
| Riboflavin | 1,6 mg |
| Pyridoxin HCl | 2,2 mg |
| Vitamin B₁₂ | 1 mcg |
| Niacin | 18 mg |
| Pantothensäure | 6 mg |
| Folsäure | 200 mcg |
| Biotin | 150 mcg |
| Cholinascorbat* | 150 mg |
| Magnesium | 100 mg |
| Zink | 15 mg |
| Mangan | 2,5 mg |
| Selen | 62 mcg |

| | |
|---|---|
| * hergestellt nach Beispiel 1 | |

### Beispiel 6

Multivitamintablette folgender Zusammensetzung:

| | |
|---|---|
| Vitamin C | 500 mg |
| Thiamin | 100 mg |
| Riboflavin | 100 mg |
| Niacin | 100 mg |
| Vitamin B₆ | 100 mg |
| Vitamin B₁₂ | 500 mcg |
| Pantothensäure | 100 mg |
| Folsäure | 400 mcg |
| Biotin | 50 mcg |
| Cholinascorbat* | 500 mg |

| | |
|---|---|
| * hergestellt nach Beispiel 1 | |

## Patentansprüche

1. Verfahren zur Herstellung von kristallinem Cholinascorbat durch Umsetzung von Ascorbinsäure mit Trimethylamin und Ethylenoxid, **dadurch gekennzeichnet, daß** die Reaktion im Temperaturbereich von 0°C bis 5°C und in Gegenwart eines mit Wasser mischbaren organischen Lösungsmittels oder einer Mischung aus Wasser und einem mit Wasser mischbaren organischen Lösungsmittel durchgeführt wird.

2. Verfahren nach Anspruch 1, **dadurch gekennzeichnet, daß** die Kristallisation von Cholinascorbat in dem für die Reaktion verwendeten Lösungsmittel erfolgt.

## Claims

1. A process for preparing crystalline choline ascorbate by reacting ascorbic acid with trimethylamine and ethylene oxide, which comprises carrying out the reaction in the temperature range from 0°C to 5°C and in a water-miscible organic solvent or a mixture of water and a water-miscible organic solvent.

2. The process according to claim 1, wherein choline ascorbate is crystallized in the solvent used for the reaction.

## Revendications

1. Procédé de préparation d'ascorbate de choline cristallin par réaction d'acide ascorbique avec de la triméthylamine et de l'oxyde d'éthylène, **caractérisé en ce que** la réaction est effectuée dans la plage de températures de 0°C à 5°C et en présence d'un solvant organique miscible à l'eau ou d'un mélange d'eau et d'un solvant organique miscible à l'eau.

2. Procédé suivant la revendication 1, **caractérisé en ce que** la cristallisation d'ascorbate de choline a lieu dans le solvant utilisé pour la réaction.
